Europäisches Patentamt

European Patent Office

Office européen des brevets

(19)

(11) Publication number: **0 293 207**
**A2**

## EUROPEAN PATENT APPLICATION

(12)

(21) Application number: **88304800.1**

(22) Date of filing: **26.05.88**

(51) Int. Cl.⁴: **C 12 N 1/20**
C 12 N 15/00, C 12 P 13/22
//(C12N1/20,C12R1:19)

(30) Priority: **29.05.87 US 55381**

(43) Date of publication of application:
**30.11.88 Bulletin 88/48**

(84) Designated Contracting States: **BE DE FR GB**

(71) Applicant: **THE STANDARD OIL COMPANY**
**200 Public Square, 36-F-3454**
**Cleveland Ohio 44114-2375 (US)**

(72) Inventor: **Kung, Wei-Jen**
**34430 Sherbrook Park Drive**
**Solon Ohio 44139 (US)**

(74) Representative: **Ryan, Edward Terrence et al**
**BP INTERNATIONAL LIMITED, Patents Division Chertsey Road**
**Sunbury-on-Thames Middlesex, TW16 7LN (GB)**

The applicant has filed a statement in accordance with Rule 28 (4) EPC (issue of a sample only to an expert). Accession number(s) of the deposit(s): ATCC 67399.

(54) **Eschericia coli carrying recombinant plasmid for the production of tryptophan.**

(57) A recombinant DNA plasmid comprising a tryptophan operon which encodes an anthrinilate synthese desensitized to feedback inhibition and an autoamplifying cloning vector and wherein said plasmid is able to replicate in a strain of Escherichia bacteria that is lacking of tryptophan operon.

EP 0 293 207 A2

## Description

# ESCHERICIA COLI CARRYING RECOMBINANT PLASMID FOR THE PRODUCTION OF TRYPTOPHAN

## BACKGROUND

The present invention is directed generally to the production of L-tryptophan from a microorganism containing a plasmid that contains an autoamplifying cloning vector and a desensitized tryptophan operon. The invention also relates to the novel plasmid replicated in an Eschericia strain that has been modified so it does not damage or prohibit the production of tryptophan.

L-tryptophan has been produced by fermentation using wild type strains or mutant strains of Escherichia coli, Bacillus subtilis and Corynebacterium glutamicum. However, conventional fermentation methods have not successfully produced high levels of the amino acid tryptophan.

It is known to use plasmids with genes encoding for products such as polypeptides and protein, by cultivating bacteria carrying a plasmid for these products. L-tryptophan has been produced from Bacillus strains containing a plasmid of DNA fragment controlling resistance to tryptophan-antagonists, see U.S. 4,588,687 to Tsuchida et al. U.S. 4,371,614 discloses the production of L-tryptophan from an Eschericia coli bacteria deficient in the tryptophan enzyme carrying a plasmid with a tryptophan operon gene. Canadian Patent No. 1,182,409 to Aiba et al. discloses a recombinant plasmid having a tryptophan operon in an Eschericia coli for the production of tryptophan. By increasing the level of expression of genes, the tryptophan can be over-expressed resulting in disturbance of the cellular physiology and a slowing down of the bacterial growth rate.

It has also been shown that the expression of genes producing a desired product such as a protein can be obtained by using an inducible promoter such as $P_L$ promoter of phage lambda, see Remaut E., P. Stanssens and W. Fiers, Plasmid Vectors for High-Efficiency Expression Controlled by the $P_L$ Promoter of Coliphage Lambda, Gene 15, 81-93, 1981. U.S. 4,499,189, U.S. 4,495,287, and U.S. 4,487,835 to Uhlin et al discloses the use of heat-inducible plasmids to produce high yields of gene products of plasmid DNA. Frey et al. discloses the use of autoamplifying cloning vectors that codes for colicin D, a protein antibiotic. The plasmid autoamplifies when the culture of the host bacteria enters the stationary phase of growth, see Frey, J. and K. N. Timmis, Col D-derived Cloning Vectors that Autoamplify In the Stationary Phase of Bacterial Growth, Gene 35, 103-111, 1985.

A need exists for microorganisms capable of producing high levels of tryptophan without affecting the growth properties of the microorganisms, by overexpression of the genes. Tryptophan productivity is a function of both cell density and gene dosage, therefore, it is advantageous to grow the microorganism to a high cell density and then increase the gene dosage by using an autoamplifying plasmid. Thus, the inventive process produces high tryptophan productivity by employing a separate growth and expression/production phase of the microorganism. Further, large scale fermentation is more viable by employing a separate growth and expression phase because induction by temperature and/or by chemicals disturb the equilibrium of the system and the cellular physiology of the microorganism.

It is an object of this invention to provide a microorganism which comprises a host of the genus Eschericia deficient in tryptophan degradation genes and carrying a plasmid with genetic information to produce tryptophan and being desensitized to tryptophan.

Another object of this invention is to provide a recombinant plasmid having an autoamplifying cloning vector and a tryptophan operon desensitized to tryptophan feedback inhibition in a strain of Eschericia bacteria for the production of L-tryptophan.

Another object of the instant invention is to provide a method for the production of L-tryptophan, in particular by fermentation which comprises culturing bacteria from the genus Eschericia containing a plasmid with genetic information to produce high levels of tryptophan which tryptophan is released into the media and recovering the L-tryptophan from the culture medium.

These and other objects, together with the advantages over known methods shall become apparent from the specification which follows and are accomplished by the invention hereinafter described and claimed.

## Summary of the Invention

The invention provides a DNA plasmid that codes for the production of the amino acid tryptophan by containing an autoamplifying cloning vector and a desensitized tryptophan operon which is transformed into an Eschericia bacteria strain lacking the necessary functions to breakdown tryptophan. The Eschericia strain with the constructed plasmid is capable of producing high levels of L-tryptophan by a fermentation process.

The invention provides a process for producing L-tryptophan comprising cultivating an Eschericia bacteria strain carrying a plasmid containing an autoamplifying cloning vector and a desensitized tryptophan operon and harvesting from the media of the bacterial culture the tryptophan product of the plasmid.

## Detailed Description of the Invention

The present invention provides a process for high levels of L-tryptophan production by fermentation employing a constructed plasmid transformed into an Eschericia coli. The plasmid is constructed by inserting tryptophan biosynthesis genes into a vector that automatically increases the plasmid copy number after the host bacteria reaches the stationary growth phase.

The recombinant plasmid of the instant invention comprises a tryptophan operon and an autoamplifying vector. The recombinant plasmid of this invention is constructed with a tryptophan operon. The operon is a coordinated unit of genetic expression for tryptophan which consists of regulatory genes of operator, promoter and attenuation for tryptophan and structural genes of trp E, trp D, trp C, trp B and trp A. The structural genes code for five enzymes of anthranilate synthase, anthranilate phosphoribosyl transferase, phosphorisosyl anthranilate isomercase, indolegycerol phosphate synthase and tryptophan synthase that convert chorismate into tryptophan.

Plasmids used in this invention contain a tryptophan operon which is desensitized to tryptophan feedback inhibition by expressing feedback resistant of anthranilate synthetase and/or anthranilate phosphoribosyl transferase so that increased levels of tryptophan can be produced.

Plasmids carrying a wild type Eschericia coli tryptophan operon DNA have been constructed using transducing phages such as lambda EA60-3, lambda ED10, lambda 080pt190 and the like, see, Deeb, S. S., K. Okamoto and B. D. Hall, Virology, 31, 289-295, 1967. Exemplary wild type tryptophan operon plasmids include but are not limited to the following plasmids and their derivatives of pVH151 plasmid, see Hershfield, V. H. W. Boyer, C. Yanofsky, M. A Lovett and D. R. Helinski Proc. Nat. Acad. Sci., U.S.A. 71,3451-3459, 1974; pVH153 plasmid see, Helinski, D. R., V. Hershfield, D. Figurski and R. J. Meyer in Recombinant Molecules: Impact on Science and Society, Raven Press, New York, pp 151-165, 1977; pSC101-trp plasmid, RSF1010-trp plasmid, RSF2124-trp plasmid, RP4-trp plasmid, see, Nagahari, K., T. Tanaka, F. Hishinuma, M. Kuroda, and K. Sakaguchi, Gene, 1, 141, 1977; and the like. The wild type tryptophan operons preferably are mutated to be desensitize to feedback inhibition.

Plasmids carrying a mutated tryptophan operon gene are prepared by known techniques, such as chemical mutation of strains containing the above-identified plasmids, direct cloning of the feedback resistant tryptophan genes in cellular DNA of mutant strains and the like. Typical examples of mutated tryptophan operon plasmids include but are not limited to pSC101-trpl15, pSC101-trpl15-14 plasmid, see Aiba, S. H. Tsunekawa and T. Imanaka, Appl. and Environ. Microbiol. 43,289 (1982), pGx14 plasmid, pGx15 plasmid, pGx16 plasmid, pGx18 plasmid, pGx19 plasmid, see D. M. Anderson, K. M. Herrmann and R. L. Somerville, U.S. Patent 4,371,614 (1983) and the like.

The tryptophan operon described above is inserted into an autoamplifying cloning vector. These vectors autoamplify when the culture of host bacteria containing the autoamplifying vectors enter stationary phase of growth of the host bacteria, resulting in a substantial increase in the expression of the tryptophan cloned gene as a consequence of the increase in gene dosage. The plasmid copy number increases from about 15 copies/chromosome in the exponential phase of bacteria host growth to about 150 copies/chromosome at the beginning of the stationary phase without temperature or chemical induction. Thus, the rate of replication of the plasmid is adjusted by the growth rate of the culture so that the expression of the tryptophan gene occurs at high levels when high host cell density has been obtained. The vector needs to have a high stability in the host Eschericia strain.

Typical autoamplifying cloning vectors include but are not limited to the following vectors and their derivatives of pKT 235, colD, ColE-1, and the like. Most preferred is pKT 235, see Frey J. & Timmis K. N. ColD-Derived Cloning Vectors That Autoamplify In the Stationary Phase of Bacterial Growth, Gene, 35 103-111 (1985).

Recombination of the tryptophan operon into the autoamplifying cloning vector yields a tryptophan (trp) autoamplifying (pKT) composite plasmid. The plasmid containing the autoamplifying cloning vector and a desensitized tryptophan operon is constructed using known recombinant DNA techniques such as restriction enzyme digestion, ligation and transformation, see Maniatis T., E. F. Fritsch and J. Sambrook, Molecular Cloning: A Laboratory Manual, Cold Spring Harbor Laboratory, 1982.

A plasmid that encodes for tryptophan can be constructed by using the known techniques. Illustrative of this is the following; the tryptophan operon is excised from bacterial chromosonal DNA, preferably from the host bacteria of Eschericia by restriction enzyme digestion techniques. The tryptophan operon is inserted into a vector by ligation techniques. The vector with the tryptophan operon is then inserted into a host strain by transformation techniques. The plasmids containing the tryptophan operon in the host strain are selected for by known selection techniques such as antibody resistance and the like.

Restriction enzyme technology employs endonucleases which recognize specific sequences within double-strand DNA and cut the DNA within or near to their particular recognition sequences, which typically are four to six nucleotides in length with a twofold axis of symmetry. The autoamplifying vector is cleaved by a suitable restriction enzyme at a unique site to form a linear DNA molecule. The desirable tryptophan operon can also be cut off from a plasmid or cellular DNA using a suitable restriction enzyme to cut out the tryptophan DNA.

Recombination of a tryptophan operon with the linearized autoamplifying vector can be carried out by a ligation reaction with a ligase such as T4 ligase and the like. Self-annealing of the vector DNA can be suppressed by dephosphorylation prior to the insertion of the tryptophan DNA fragment by treating with alkaline phosphatase, see F. Bolivar and K. Backman in Methods in Enzymology, S. P. Colowick and N. O. Kaplan eds., vol 68, p. 245, Academic Press, New York, 1979.

The plasmid of the present invention is transformed into an Eschericia host by known techniques such as calcium shock, rubidium chloride and calcium chloride shock, heat shock and the like. A shock procedure makes the hosts competent for DNA uptake, see Morrison, D. A., J. Bact, 132, 349-351, 1977. The host cells are grown to log phase in a rich medium, washed, treated with a buffer containing about 100 mM CaCl₂, and then exposed to a ligation mixture which contains the recombined plasmid. The plasmid is transformed into the host cells. Antibiotic markers, that is, DNA that codes for antibiotic resistance on the plasmid, the tryptophan gene marker or the like can be used to select the desired transformation isolates by known plating techniques.

The host strain is any Eschericia coli bacteria. The host strain can be obtained by conventional mutation techniques. Recipient microorganism for the hybrid DNA plasmids include all Eschericia coli strains, preferably any Eschericia coli strains that are derived from E. coli K12.

Eschericia coli host strains have the following desirable characteristics for enhanced production of tryptophan; defective tryptophan repressor enzyme (trpR), tryptophanase deficient (tnaA), and/or tryptophan operon-lacking strains (ΔtrpEA). Preferably the host strain contains more than one of the aforementioned characteristics in order to maximize the biosynthetic flow and production of tryptophan. These properties are described below and can be obtained by conventional mutation techniques.

Tryptophan repressor deficient mutation (trpR) can be obtained from Eschericia coli strains. TrpR gene produces a protein, aporepressor which at high tryptophan concentration complexes with tryptophan to form an active repressor. The complexed repressor binds tightly to the operator of tryptophan operon, blocks the transcription of the trp genes and therefore the production of tryptophan-synthesis enzymes. For a tryptophan producing strain it is preferrable that this gene is mutated so that the pathway proceeds derepressed regardless of the level of tryptophan. Tryptophan repressor mutants can be selected from mutants that are resistant to tryptophan analogs such as 5- methyl-tryptophan and 6-methyl tryptophan. Examples can be found in J. Mol. Biol., 44 p. 185-193, 1969 and Genetics 52 p. 1303-1316, 1965.

Tryptophanase-deficient mutation (tnaA) of Eschericia coli can be obtained from all wild type Eschericia coli strains. The tryptophanase degrades tryptophan to give indole, pyruvic acid and ammonia. For high tryptophan production, it is preferrable that this enzyme activity is deficient to ensure that the tryptophan produced is not broken down. The mutation and selection of this type of mutant is based on the inability of the mutant to use tryptophan as a carbon source. A detailed example can be found in J. Bacteriology, 89, p. 680-686, 1965.

Tryptophan operon deletion mutation (ΔtrpEA) of Eschericia coli can be obtained in Eschericia coli mutants such as W3110 ΔtrpEA1 and W3110 ΔtrpEA2 developed by Dr. C. Yanofsky at Stanford University. For detailed description of the production of such deletion mutants, see J. Spudich, V. Horn and C. Yanofsky J. Mol. Biol. 53 p. 49-67, 1970, and Canadian patent 1,182,409 to Aiba et al.

The transformed Eschericia coli with the autoamplifying cloning vector and the desensitized tryptopyhan operon is cultivated under aerobic conditions, in which the pH of the medium is adjusted to about 6 to about 8, preferably around 7 and the temperature of cultivation is about 20°C to about 50°C, preferably about 30°C to about 40°C. Conventional methods are employed for culturing the L-tryptophan producing Eschericia coli strain and are similar to methods for the cultivation of known L-tryptophan producing microorganisms. The medium for cultivating the microorganisms may be any natural or synthetic nutrient medium. The culture medium employed contains carbon sources, nitrogen sources, inorganic materials and if required minor organic nutrients such as vitamins, amino acids, and/or antibiotics. Suitable carbon sources include but are not limited to glucose, lactose, starch hydrolysate, molasses, fructose, glycerol, maltose and the like. Suitable nitrogen sources include but are not limited to gaseous ammonia, aqueous ammonia and ammonium salts and other nitrogen containing materials. Examples of inorganic materials include but are not limited to potassium monohydrogen phosphate, potassium dihydrogen phosphate, magnesium sulfate, sodium chloride, ferrous sulfate, calcium chloride, zinc chloride, copper sulfate, manganese chloride, cobalt chloride, ammonium molybdate, boric acid and the like.

The transformed Eschericia coli releases the tryptophan into the media, which tryptophan is removed by conventional separation techniques such as ion exchange resin, activated carbon, concentration and precipitation and the like.

## Specific Embodiments

Having generally described this invention, a further understanding can be obtained by reference to certain specific examples which are provided herein for the purpose of illustration only and they are not intended to be limiting. Further, it is understood that variations and modifications can be made by one skilled in the art without departing from the spirit and scope of the invention.

## Isolation of the Desensitized Tryptophan Gene Operon

E. coli W3110 ΔtrpEA1 trp R tna A (pSC101 trpI15) obtained from American Type Culture Collection No. 31743, Rockville, Maryland, was innoculated into Luria broth containing about 10 grams of tryptone, about 5 grams of yeast extract and about 5 grams of sodium chloride in one liter of water. The E. coli was cultured at about 37°C overnight with shaking. The E. coli bacterial cells were harvested by centrifugation and then the cell were lysed by lyzozymes/alkoline SDS (sodium dodecysulfate) treatment. This mixture was then treated with sodium acetate solution and centrifuged at about 15000 rpms for about 30 minutes. The resultant clear supernatant liquid was treated with an equal volume of phenol to remove precipitated proteins. The DNA was then precipitated and recovered from the aqueous solution by addition of two volumes of absolute (100%) ethanol. The recovered DNA was redissolved and loaded onto a cesium chloride gradient containing ethidium bromide of about 600 ug/ml which was then centrifuged at about 45000 rpms for about 24 hours resulting in two bands. The lower band was recovered and washed with equal volume of butanol to removed the ethidium bromide. The lower band was then treated with ethanol to precipitate the plasmid pSC101 trpl15. This plasmid then was digested by restriction enzyme EcoRl into two fragments of the vector pSC101 and the tryptophan trpl15. The tryptophan operon DNA was stored in about a -20°C freezer.

Isolation of the Autoamplifying Cloning Vector

E. coli C600 obtained from Prof. Sommerville from Purdue University containing a autoamplifying (pKT235) plasmid was innoculated into about 10ml of Luria broth overnight at about 37°C. The cells were harvested by centrifugation and then suspended in about 100 microliters of GET buffer of about 50mM glucose, about 25mM tris buffer, about 10mM EDTA with a pH of about 8. The cells suspended in the GET buffer were treated with lyzozyme/alkaline SDS treatment. The protein was precipitated by the addition of about 3M sodium acetate solution. This mixture was centrifuged for about 5 minutes in an Eppendorf microcentrifuge to remove the cells. Crude autoamplifying plasmid was obtained by adding two volumes of ethanol to the supernatant. The DNA was then resuspended in about 0.3M sodium acetate and reprecipitated by adding two volumes of ethanol to the suspension. The DNA was stored in about 15 microliters of low TE buffer of 1 millimolar tris, 0.1 mM EDTA at about a pH of 8. This plasmid has one linear EcoRl site and a chloramphenicol, kanamycin and/or ampicillin resistant gene regions.

Ligation of the Autoamplifying Cloning Vector (pKT235) and the Tryptophan Operon (trpl15)

About 10 µg of pSC101 trpl15 and about 1mg of pKT 235 were each treated separately for about 2 hours at about 37°C with restriction enzyme EcoRl. Agarose gel electrophoresis was used to ensure complete digestion by enzymatic reaction. The reaction mixtures were each treated with phenol to remove excess enzyme. Two volumes of ethanol was added to each mixture to precipitate the DNA and the DNA was resuspended in a low molar Tris/EDTA buffer.

About 3 µg of fragmented tryptophan operon plasmid (pSC101 trpl15) and about 0.2 µg of digested autoamplifying plasmid (pKT235) were mixed together in about 20 µl of ligation buffer of about 50mM tris, about 10mM MgCl2, about 1mM spermidine, about 10mM DTT, about 1mM ATP and about 0.1mg/ml BSA with a pH of about 7.4. One unit of T4 DNA ligate was added into the mixture and the mixture was incubated overnight at about 16°C. The ligation mixture was stored in about a -80°C freezer before use in cloning experiments.

Transformation of E. coli EA2 With The New Plasmid

E. coli EA2 (E. coli W3110 tnaA2 ΔtrpEA2 was employed as the host organism and was obtained from Dr. C. Yannovsky at Stanford University. The E. coli EA2 strain is tryptophan operon deficient and requires tryptophan supplement for growth.

About 5 ml of Luria broth was inoculated with 0.6 ml of an overnight culture of E. coli EA2 and the mixture incubated at 37°C for one hour. The cells are pelleted by centrifugation at 3000 rpm. The centrifuged pellet was washed with sterile about 10mM sodium chloride solution and resuspended in about 10ml of calcium chloride solution of about 100mM CaCl2 in about 20mM Tris, at about 0°C for about 30 minutes. The mixture was then centrifuged and the pellet resuspended in about 1ml of calcium chloride solution. About 0.2ml of this suspension was added to about 4µl of the ligation mixture (from the above paragraph) in about 0.15ml of calcium chloride solution. The mixture was left on ice for about 1 1/2 hours. Then about 6ml of Luria broth was added to the mixture and incubated for about 1 1/2 hours at about 37°C.

This mixture was then plated as follows: The cells were first plated on Luria broth agar plates supplemented with about 20mg/ml of ampicillin. The plates were incubated overnight at about 37°C. The colonies grown overnight were transferred onto three different Luria agar plates; one supplemented with about 20mg/ml of ampicillin, one supplemented with about 20mg/ml ampicillin and about 30 mg/ml chloramphenicol and the third supplemented with about 20mg/ml of ampicillin and about 10mg/ml of tetracycline. Colonies which grew on the ampicillin plate but not on the ampicillin and chloramphenicol plate or the ampicillin and tetracycline plate were selected. One such colony was designated E. coli EA2 (pKTtrp20), that, is E. coli host strain carrying the autoamplifying cloning vector with the tryptophan operon.

Results

The copy number of pkTtrp20 in E. coli EA2 at stationary phase was estimated to be about 50 to 100 copies per chromosome by plasmid DNA quantitation.

E. coli EA2 had its tryptophan gene deleted from the chromosomal DNA, therefore it cannot grow on M9 minimal medium without tryptophan supplements. The strain of the instant invention harboring the novel autoamplifying tryptophan operon-plasmid grew on the M9 minimal medium. Further the novel strain was grown in M9 minimal broth overnight at about 37°C. The culture was tested in a spectrograph and showed an O.D. (optical density) of 1.0. Therefore, this demonstrates that the modified E. coli EA2 strain contains the autoamplifying vector with the tryptophan operon.

A frozen stock culture of the E. coli EA2(pKTtrp20) strain obtained by transforming EA2 strain with pKT trp20 was used to inoculate about 5 mL of Luria broth containing about 35 ug/mL ampicillin and precultivate at about 37°C for about 5 to 6 hours. Then about 0.5 mL of the preculture was inoculated into about 20-40 mL of medium MT containing per liter about 3 g of $KH_2PO_4$, about 7 g of $K_2HPO_4$, about 3 g of $NH_4Cl$, about 10 mg of $FeSO_4$ $7H_2O$, about 0.2 g of $MgSO_4$ $7H_2O$, about 30.0 g of glucose, about 3 g of casamino acid, about 0.8 g of anthranilic acid, about 35mg ampicillin and 40 g of $CaCO_3$ wherein pH was adjusted to about 7.0 using $NH_4OH$, and cultivated on a shaker at about 37°C and about 200 rpm after about 24 hrs. The tryptophan in the MT medium was assayed by high pressure liquid chromatography method using a reverse phase C18 column. The assay demonstrated about 1 to about 1.2g/L of tryptophan production.

By the process of the instant invention high levels of L-tryptophan are produced using the Eschericia coli host strain containing a plasmid of an autoamplifying cloning vector and a desensitized tryptophan operon.

The Eschericia coli bacteria containing the recombinant plasmid of an autoamplifying cloning vector and tryptophan operon was deposited with the America Type Culture Collection (ATCC) in Rockville, Maryland, under agreement whereby they would become accessible to the public upon the issuance of Letters Patent. No. 67399 is assigned to the bacteria culture deposited with the ATCC.

Although the invention has been described in detail through the preceeding examples, these examples are for the purpose of illustration only and it is understood that variations and modifications can be made without departing from the spirit and scope of the invention.

**Claims**

1. A strain of Eschericia coli W3110 trpEA2 tnaA and is identified as ATCC 67399 carrying a recombinant plasmid having an autoamplifying cloning vector and desensitized tryptophan operon.

2. The strain of Eschericia coli of claim 1 wherein the Eschericia coli has at least one of a defective tryptophan repressor enzyme, is tryptophanase deficient or has a defective tryptophan operon lacking strain.

3. The plasmid of claim 1 wherein said plasmid's tryptophan operon is desensitized to tryptophan feedback inhibition.

4. A recombinant plasmid that codes for the production of tryptophan, containing an autoamplifying cloning vector and a desensitized tryptophan operon and which recombinant plasmid is transformed into and able to replicate in a strain of Eschericia coli.

5. The recombinant plasmid of claim 4 selected from the group consisting of pKT 235, ColD, colE-1 and combinations thereof and which is transformed into and able to replicate in Eschericia coli trp I15 pKT 235.

6. A process to produce tryptophan comprising cultivating an Eschericia bacteria strain wherein said Eschericia coli strain is Eschericia coli W 3110 trpEA2 tnaA (pKTtrp20) and is identified as ATCC 67399 and carrying a plasmid containing an autoamplifying cloning vector and a desensitized tryptophan operon and harvesting from the media the tryptophan product of the plasmid.

7. The process of claim 6 wherein said plasmid codes for the production of tryptophan and the tryptophan operon is desensitized to tryptophan feedback inhibition by expressing feedback resistant of anthranilate synthetase, anthranilate phosphorisosyl transferase and combinations thereof.

8. The process of claim 6 wherein said Eschericia coli strain has at least one of a defective tryptophan repressor enzyme, is tryptophanase deficient or has a defective tryptophan operon lacking strain.